# EUROPEAN PATENT SPECIFICATION

(11) **EP 3 370 670 B1**
(45) Date of publication and mention of the grant of the patent: **30.03.2022**
(21) Application number: 16794903.1
(22) Date of filing: 04.11.2016
(51) Int. Cl.: A61F 13/534, A61F 13/53

(54) **FOAM ABSORBENT CORE STRUCTURE COMPRISING HETEROGENEOUS MASS**
SCHAUMABSORBIERENDE KERNSTRUKTUR MIT HETEROGENER MASSE
STRUCTURE CENTRALE DE MOUSSE ABSORBANTE COMPRENANT UNE MASSE HÉTÉROGÈNE

(30) Priority: 04.11.2015 US 201562250810 P
(43) Date of publication of application: 12.09.2018
(73) Proprietor: The Procter & Gamble Company, Cincinnati, OH 45202 (US)
(72) Inventor: BEWICK-SONNTAG, Christopher, Philip, Cincinnati, Ohio 45202 (US); KIRKBRIDE, Tana, Marie, Cincinnati, Ohio 45202 (US); HUBBARD JR., Wade, Monroe, Cincinnati, Ohio 45202 (US)
(74) Representative: P&G Patent Germany
(86) International application number: PCT/US2016/060590
(87) International publication number: WO 2017/079603

(56) References cited:
- WO-A1-01/21227
- WO-A1-2013/180937
- WO-A1-2016/160900
- US-A1- 2001 024 716
- US-B1- 6 673 981

## Description

### FIELD OF THE INVENTION

The present invention relates to an absorbent structure utilizing a plurality of absorbent core layers. The absorbent core structure is useful in absorbent articles such as diapers, incontinent briefs, training pants, diaper holders and liners, sanitary hygiene garments, and the like. Specifically, the present invention relates to an absorbent core structure that utilizes a heterogeneous mass layer comprising open-cell foam.

### BACKGROUND OF THE INVENTION

Open-celled foams are used for their absorbent properties. Open-celled foams include latex polymer foams, polyurethane foams, and foams created by polymerizing an emulsion. One type of an open-celled foam is created from an emulsion that is a dispersion of one liquid in another liquid and generally is in the form of a water-in-oil mixture having an aqueous or water phase dispersed within a substantially immiscible continuous oil phase. Water-in-oil (or oil in water) emulsions having a high ratio of dispersed phase to continuous phase are known in the art as High Internal Phase Emulsions, also referred to as "HIPE" or HIPEs. Different foams may be chosen due to specific properties.

Traditionally, open-celled foams are polymerized in a continuous sheet or in a tubular reaction. Either process represents that one must use polymerized open-celled foam in a continuous form or break up the polymerized open-celled foam to make open-celled foam pieces.

Ultimately, in regards to an absorbent core, the current process represents using a core made solely of foam or a core that uses pieces of foam placed into or onto another material. This means that the pieces must be held in place by a cover layer or some form of adhesive. The process does not allow one to make an absorbent core wherein discrete portions of the foam are integrated into a substrate and parts of the substrate are integrated into the foam. Further, the process creates a uniform foam that does not allow for different types of pores in terms of size magnitude differences.

WO2016/160900 discloses a plurality of heterogenous masses layered so that the first heterogeneous mass may have foam particles enrobing a nonwoven while a second heterogeneous mass adjacent the first heterogeneous mass may have foam particles enrobing a film or one surface of a film. The open cell foam is a thermoset polymeric foam made from the polymerization of a High Internal Phase Emulsion (HIPE), also referred to as a polyHIPE.

Therefore there exists a need to create a heterogeneous mass containing foam. The foam may be integrated into the heterogeneous mass by enrobing enrobeable elements within the heterogeneous mass. The heterogeneous mass structure comprising open-cell foam may be combined in an absorbent core structure with other absorbent layers to create the absorbent core structure.

### SUMMARY OF THE INVENTION

The invention is for an absorbent product as indicated in claim 1, the absorbent product comprising a topsheet, a backsheet, and an absorbent core structure, wherein the absorbent core structure comprises of at least two layers. A top core layer comprises a heterogeneous mass comprising a longitudinal axis, a lateral axis, a vertical axis, and a HIPE foam intermixed with a nonwoven web. The heterogeneous mass contains one or more stripes of high capillarity and one or more canals of high permeability. The stripes of high capillarity contain a HIPE foam and nonwoven web. The canals of high permeability contain between 0% and 40% of the amount of HIPE foam in the stripes of high capillarity and nonwoven web. A lower core level comprises a substrate plus superabsorbent polymer layer containing greater than 50%, of a superabsorbent polymer and less than 30% of cellulose.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
FIG. 1 is a perspective view of one embodiment of a sanitary napkin.
FIG. 2 is a cross-sectional view of the sanitary napkin of Fig. 1, taken through line 2-2.
FIG. 3 is a cross-sectional view of the sanitary napkin of Fig. 1, taken through line 3-3.
FIG. 4 is an SEM micrograph of a heterogeneous mass.
FIG. 5 is an SEM micrograph of a heterogeneous mass.
FIG. 6 is a top view of an alternative pattern.
FIGS. 7A-C show top views of alternative patterns.
FIGS. 8A-C show top views of alternative patterns.
FIG. 9 relates to the SABAP apparatus.
FIGS. 10A-B relates to the SABAP apparatus.

### DETAILED DESCRIPTION OF THE INVENTION

As used herein, the term "absorbent core structure" refers to an absorbent core that is has two or more absorbent core layers. Each absorbent core layer is capable of retaining fluid.

As used herein, the term "bicomponent fibers" refers to fibers which have been formed from at least two different polymers extruded from separate extruders but spun together to form one fiber. Bicomponent fibers are also sometimes referred to as conjugate fibers or multicomponent fibers. The polymers are arranged in substantially constantly positioned distinct zones across the cross-section of the bicomponent fibers and extend continuously along the length of the bicomponent fibers. The configuration of such a bicomponent fiber may be, for example, a sheath/core arrangement wherein one polymer is surrounded by another, or may be a side-by-side arrangement, a pie arrangement, or an "islands-in-the-sea" arrangement.

As used herein, the term "biconstituent fibers" refers to fibers which have been formed from at least two polymers extruded from the same extruder as a blend. Biconstituent fibers do not have the various polymer components arranged in relatively constantly positioned distinct zones across the cross-sectional area of the fiber and the various polymers are usually not continuous along the entire length of the fiber, instead usually forming fibrils which start and end at random. Biconstituent fibers are sometimes also referred to as multiconstituent fibers.

The term "disposable" is used herein to describe articles, which are not intended to be laundered or otherwise restored or reused as an article (i.e. they are intended to be discarded after a single use and possibly to be recycled, composted or otherwise disposed of in an environmentally compatible manner). The absorbent article comprising an absorbent structure according to the present invention can be for example a sanitary napkin or a panty liner. The absorbent structure of the present invention will be herein described in the context of a typical absorbent article, such as, for example, a sanitary napkin. Typically, such articles can comprise a liquid pervious topsheet, a backsheet and an absorbent core intermediate the topsheet and the backsheet.

As used herein, an "enrobeable element" refers to an element that may be enrobed by the foam. The enrobeable element may be, for example, a fiber, a group of fibers, a tuft, or a section of a film between two apertures. It is understood that other elements are contemplated by the present invention.

A "fiber" as used herein, refers to any material that can be part of a fibrous structure. Fibers can be natural or synthetic. Fibers can be absorbent or non-absorbent.

A "fibrous structure" as used herein, refers to materials which can be broken into one or more fibers. A fibrous structure can be absorbent or adsorbent. A fibrous structure can exhibit capillary action as well as porosity and permeability.

As used herein, the term "meltblowing" refers to a process in which fibers are formed by extruding a molten thermoplastic material through a plurality of fine, usually circular, die capillaries as molten threads or filaments into converging high velocity, usually heated, gas (for example air) streams which attenuate the filaments of molten thermoplastic material to reduce their diameter. Thereafter, the meltblown fibers are carried by the high velocity gas stream and are deposited on a collecting surface, often while still tacky, to form a web of randomly dispersed meltblown fibers.

As used herein, the term "monocomponent" fiber refers to a fiber formed from one or more extruders using only one polymer. This is not meant to exclude fibers formed from one polymer to which small amounts of additives have been added for coloration, antistatic properties, lubrication, hydrophilicity, etc. These additives, for example titanium dioxide for coloration, are generally present in an amount less than about 5 weight percent and more typically about 2 weight percent.

As used herein, the term "non-round fibers" describes fibers having a non-round cross-section, and includes "shaped fibers" and "capillary channel fibers." Such fibers can be solid or hollow, and they can be tri-lobal, delta-shaped, and are preferably fibers having capillary channels on their outer surfaces. The capillary channels can be of various cross-sectional shapes such as "U-shaped", "H-shaped", "C-shaped" and "V-shaped". One practical capillary channel fiber is T-401, designated as 4DG fiber available from Fiber Innovation Technologies, Johnson City, TN. T-401 fiber is a polyethylene terephthalate (PET polyester).

As used herein, the term "nonwoven web" refers to a web having a structure of individual fibers or threads which are interlaid, but not in a repeating pattern as in a woven or knitted fabric, which do not typically have randomly oriented fibers. Nonwoven webs or fabrics have been formed from many processes, such as, for example, meltblowing processes, spunbonding processes, spunlacing processes, hydroentangling, airlaying, and bonded carded web processes, including carded thermal bonding. The basis weight of nonwoven fabrics is usually expressed in grams per square meter (gsm). The basis weight of the laminate web is the combined basis weight of the constituent layers and any other added components. Fiber diameters are usually expressed in microns; fiber size can also be expressed in denier, which is a unit of weight per length of fiber. The basis weight of laminate webs suitable for use in an article of the present invention can range from 10 gsm to 100 gsm, depending on the ultimate use of the web.

As used herein, the term "polymer" generally includes, but is not limited to, homopolymers, copolymers, such as for example, block, graft, random and alternating copolymers, terpolymers, etc., and blends and modifications thereof. In addition, unless otherwise specifically limited, the term "polymer" includes all possible geometric configurations of the material. The configurations include, but are not limited to, isotactic, atactic, syndiotactic, and random symmetries.

As used herein, "spunbond fibers" refers to small diameter fibers which are formed by extruding molten thermoplastic material as filaments from a plurality of fine, usually circular capillaries of a spinneret with the diameter of the extruded filaments then being rapidly reduced. Spunbond fibers are generally not tacky when they are deposited on a collecting surface. Spunbond fibers are generally continuous and have average diameters (from a sample size of at least 10 fibers) larger than 7 microns, and more particularly, between about 10 and 40 microns.

As used herein, a "strata" or "stratum" relates to one or more layers wherein the components within the stratum are intimately combined without the necessity of an adhesive, pressure bonds, heat welds, a combination of pressure and heat bonding, hydro-entangling, needlepunching, ultrasonic bonding, or similar methods of bonding known in the art such that individual components may not be wholly separated from the stratum without affecting the physical structure of the other components. The skilled artisan should understand that while separate bonding is unnecessary between the strata, bonding techniques could be employed to provide additional integrity depending on the intended use.

As used herein, a "tuft" or chad relates to discrete integral extensions of the fibers of a nonwoven web. Each tuft can comprise a plurality of looped, aligned fibers extending outwardly from the surface of the web. In another embodiment each tuft can comprise a plurality of non-looped fibers that extend outwardly from the surface of the web. In another embodiment, each tuft can comprise a plurality of fibers which are integral extensions of the fibers of two or more integrated nonwoven webs.

While particular embodiments of the present invention have been illustrated and described, it would be obvious to those skilled in the art that various other changes and modifications can be made without departing from the spirit and scope of the invention.

### GENERAL SUMMARY

An absorbent core structure is disclosed. The absorbent core structure has two or more absorbent core layers. The absorbent core layers may be joined or separate. In an embodiment, one of the absorbent core layers is a heterogeneous mass layer comprising one or more enrobeable elements and one or more discrete open-cell foam pieces.

The absorbent core structure is a two layer system wherein the top core layer is a heterogeneous mass layer comprising one or more enrobeable elements and one or more discrete open-cell foam pieces. The top core layer heterogeneous mass layer may be a stratum as defined above. The lower core level is an absorbent layer that comprises superabsorbent polymer. The absorbent core structure may comprise additional layers below the absorbent layer that comprises superabsorbent polymer.

The absorbent core structure may comprise a heterogeneous mass layer as those described in US patent application no. 61/988,565, filed May 5, 2014; US patent application no. 62/115,921, filed February 13, 2015; or US patent application no. 62/018,212. The heterogeneous mass layer has a depth, a width, and a height.

The one or more discrete portions of foam pieces enrobe the elements. The discrete portions of foam pieces are open-celled foam. In an embodiment, the foam is a High Internal Phase Emulsion (HIPE) foam.

In the following description of the invention, the surface of the article, or of each component thereof, which in use faces in the direction of the wearer is called wearer-facing surface. Conversely, the surface facing in use in the direction of the garment is called garment-facing surface. The absorbent article of the present invention, as well as any element thereof, such as, for example the absorbent core, has therefore a wearer-facing surface and a garment-facing surface.

The heterogeneous mass layer contains one or more discrete open-cell foam pieces foams that are integrated into the heterogeneous mass comprising one or more enrobeable elements integrated into the one or more open-cell foams such that the two may be intertwined.

The open-cell foam pieces may comprise between 1% of the heterogeneous mass by volume to 99% of the heterogeneous mass by volume, such as, for example, 5% by volume, 10% by volume, 15% by volume, 20% by volume, 25% by volume, 30% by volume, 35% by volume, 40% by volume, 45% by volume, 50% by volume, 55% by volume, 60% by volume, 65% by volume, 70% by volume, 75% by volume, 80% by volume, 85% by volume, 90% by volume, or 95% by volume.

The heterogeneous mass layer may have void space found between the enrobeable elements, between the enrobeable elements and the enrobed elements, and between enrobed elements. The void space may contain gas. The void space may represent between 1% and 95% of the total volume for a fixed amount of volume of the heterogeneous mass, such as, for example, 5%, 10%, 15%, 20%, 25%, 30%, 35%, 40%, 45%, 50%, 55%, 60%, 65%, 70%, 75%, 80%, 85%, 90% of the total volume for a fixed amount of volume of the heterogeneous mass.

The combination of open-cell foam pieces and void space within the heterogeneous mass may exhibit an absorbency of between 10 g/g to 200 g/g of the heterogeneous mass, such as for example, 40 g/g, 60 g/g, 80 g/g, 100 g/g, 120 g/g, 140 g/g 160 g/g 180 g/g or 190 g/g of the heterogeneous mass. Absorbency may be quantified according to the EDANA Nonwoven Absorption method 10.4-02.

The open-cell foam pieces are discrete foam pieces intertwined within and throughout a heterogeneous mass such that the open-cell foam enrobes one or more of the enrobeable elements such as, for example, fibers within the mass. The open-cell foam may be polymerized around the enrobeable elements.

In an embodiment, a discrete open-cell foam piece may enrobe more than one enrobeable element. The enrobeable elements may be enrobed together as a bunch. Alternatively, more than one enrobeable element may be enrobed by the discrete open-cell foam piece without contacting another enrobeable element.

In an embodiment, the open-cell foam pieces may enrobe an enrobeable element such that the enrobeable element is enrobed along the enrobeable elements axis for between 5% and 95% of the length along the enrobeable element's axis. For example, a single fiber may be enrobed along the length of the fiber for a distance greater than 50% of the entire length of the fiber. In an embodiment, an enrobeable element may have between 5% and 100% of its surface area enrobed by one or more open-cell foam pieces.

In an embodiment, two or more open-cell foam pieces may enrobe the same enrobeable element such that the enrobeable element is enrobed along the enrobeable elements axis for between 5% and 100% of the length along the enrobeable element's axis.

The open-cell foam pieces enrobe the enrobeable elements such that a layer surrounds the enrobeable element at a given cross section. The layer surrounding the enrobeable element at a given cross section may be between 0.01 mm to 100 mm such as, for example, 0.1 mm, 0.2 mm, 0.3 mm, 0.4 mm, 0.5 mm, 0.6 mm, 0.7 mm, 0.8 mm, 0.9 mm, 1.0 mm, 1.2 mm, 1.4 mm, 1.6 mm, 1.8 mm, 2.0 mm, 2.2 mm, 2.4 mm, 2.6 mm, 2.8 mm, or 3 mm. The layer may not be equivalent in dimension at all points along the cross section of the enrobeable element. For example, in an embodiment, an enrobeable element may be enrobed by 0.5 mm at one point along the cross section and by 1.0 mm at a different point along the same cross section.

The open-cell foam pieces are considered discrete in that they are not continuous throughout the entire heterogeneous mass layer. Not continuous throughout the entire heterogeneous mass layer represents that at any given point in the heterogeneous mass layer, the open-cell absorbent foam is not continuous in at least one of the cross sections of a longitudinal, a vertical, and a lateral plane of the heterogeneous mass layer. In a non-limiting embodiment, the absorbent foam is not continuous in the lateral and the vertical planes of the cross section for a given point in the heterogeneous mass layer. In a non-limiting embodiment, the absorbent foam is not continuous in the longitudinal and the vertical planes of the cross section for a given point in the heterogeneous mass layer. In a non-limiting embodiment, the absorbent foam is not continuous in the longitudinal and the lateral planes of the cross section for a given point in the heterogeneous mass layer.

In an embodiment wherein the open-cell foam is not continuous in at least one of the cross sections of the longitudinal, the vertical, and the lateral plane of the heterogeneous mass, one or both of either the enrobeable elements or the open-cell foam pieces may be bi-continuous throughout the heterogeneous mass.

The open-cell foam pieces may be located at any point in the heterogeneous mass. In a non-limiting embodiment, a foam piece may be surrounded by the elements that make up the enrobeable elements. In a non-limiting embodiment a foam piece may be located on the outer perimeter of the heterogeneous mass such that only a portion of the foam piece is entangled with the elements of the heterogeneous mass.

In a non-limiting embodiment, the open-cell foam pieces may expand upon being contacted by a fluid to form a channel of discrete open-cell foam pieces. The open-cell foam pieces may or may not be in contact prior to being expanded by a fluid.

An open-celled foam may be integrated onto the enrobeable elements prior to being polymerized. In a non-limiting embodiment the open-cell foam pieces may be partially polymerized prior to being impregnated into or onto the enrobeable elements such that they become intertwined. After being impregnated into or onto the enrobeable elements, the open-celled foam in either a liquid or solid state are polymerized to form one or more open-cell foam pieces.

The open cell foam pieces may be impregnated prior to polymerization into or onto two or more different enrobeable elements that are combined to create a heterogeneous mixture of enrobeable elements. The two or more different enrobeable elements may be intertwined such that one enrobeable element may be surrounded by multiples of the second enrobeable element, such as, for example by using more than one type of fiber in a mixture of fibers or by coating one or more fibers with surfactant. The two or more different enrobeable elements may be layered within the heterogeneous mass along any of the vertical, longitudinal, and/or lateral planes such that the enrobeable elements are profiled within the heterogeneous mass for an enrobeable element inherent property or physical property, such as, for example, hydrophobicity, fiber diameter, fiber or composition. It is understood that any inherent property or physical property of the enrobeable elements listed is contemplated herein.

The open-celled foam may be polymerized using any known method including, for example, heat, UV, and infrared. Following the polymerization of a water in oil open-cell foam emulsion, the resulting open-cell foam is saturated with aqueous phase that needs to be removed to obtain a substantially dry open-cell foam. Removal of the saturated aqueous phase or dewatering may occur using nip rollers, and vacuum. Utilizing a nip roller may also reduce the thickness of the heterogeneous mass such that the heterogeneous mass will remain thin until the open-cell foam pieces entwined in the heterogeneous mass are exposed to fluid.

The open-cell foam pieces may enrobe the enrobeable elements in a manner that creates a spacing or vacuole between the enrobing foam and the enrobeable element. The vacuole contains the enrobeable element and may surround the entire element, a cross section of the element, or a portion of the element. In an embodiment, the open-cell foam pieces may be in direct contact with the element at one location and spaced by a vacuole in another. The vacuole may allow the enrobeable element to move within the vacuole. The size of the vacuole may be driven by the type of enrobeable element. In an embodiment, the vacuole diameter is greater than the fiber diameter which is greater than the foam pore size. The vacuole diameter may be, for example, between 1.0001 and 30,000 times the diameter of the fiber diameter, such as, between 1.2 and 20,000 times the diameter of the fiber, 10 and 10,000 times the diameter of the fiber, 100 and 1,000, such as, for example, 20times the diameter of the fiber, 150 times the diameter of the fiber, 1,500 times the diameter of the fiber, 3,000 times the diameter of the fiber, 4,500 times the diameter of the fiber, 6,000 times the diameter of the fiber, 7,500 times the diameter of the fiber, 9,000 times the diameter of the fiber, 12,000 times the diameter of the fiber, 15,000 times the diameter of the fiber, 18,000 times the diameter of the fiber, 21,000 times the diameter of the fiber, 24,000 times the diameter of the fiber, 27,000 , or 29,000 times the diameter of the fiber.

In an embodiment, one or more vacuoles may be irregularly shaped. In such embodiments, the cross-sectional surface area of the vacuoles may be between 1.0002 and 900,000,000 times the surface area created by a cross section of the fiber. When more than one fiber is located in the same vacuole, the cross-sectional surface area of the vacuoles may be between 1.0002 and 900,000,000 times the surface area created by the sum of the cross section of the fibers, such as, for example, between 10 to 100,000,000 times the surface area created by the sum of the cross section of the fibers, between 1,000 to 1,000,000 times the surface area created by the sum of the cross section of the fibers, or between 10,000 to 100,000 times the surface area created by the sum of the cross section of the fibers.

In an embodiment, the cross-sectional surface area of the vacuoles may be between 1.26 and 9,000,000 times the cross-sectional surface area of the pores in the open-cell foam such as, for example between 100 and 5,000,000 times the cross-sectional surface area of the pores in the open-cell foam, between 1,000 and 1,000,000 times the cross-sectional surface area of the pores in the open-cell foam, between 100,000 and 500,000 times the cross-sectional surface area of the pores in the open-cell foam. The cross sectional area of the pores may be between 0.001% and 99.99% of the cross sectional area of the vacuoles. The cross-sectional surface area of the vacuoles, pores (also referred to as cells) of the open-cell foams, and fiber diameters are measured via quantitative image analysis of cross-sectional micrographs of the heterogeneous mass.

Dependent upon the desired foam density, polymer composition, specific surface area, or pore size (also referred to as cell size), the open-celled foam may be made with different chemical composition, physical properties, or both. For instance, dependent upon the chemical composition, an open-celled foam may have a density of 0.0010 g/cc to about 0.25 g/cc. Preferred 0.04 g/cc.

Open-cell foam pore sizes may range in average diameter of from 1 to 800 µm, such as, for example, between 50 and 700 µm, between 100 and 600 µm, between 200 and 500 µm, between 300 and 400 µm.

In some embodiments, the foam pieces have a relatively uniform cell size. For example, the average cell size on one major surface may be about the same or vary by no greater than 10% as compared to the opposing major surface. In other embodiments, the average cell size of one major surface of the foam may differ from the opposing surface. For example, in the foaming of a thermosetting material it is not uncommon for a portion of the cells at the bottom of the cell structure to collapse resulting in a lower average cell size on one surface.

The foams produced from the present invention are relatively open-celled. This refers to the individual cells or pores of the foam being in substantially unobstructed communication with adjoining cells. The cells in such substantially open-celled foam structures have intercellular openings or windows that are large enough to permit ready fluid transfer from one cell to another within the foam structure. For purpose of the present invention, a foam is considered "open-celled" if at least about 80% of the cells in the foam that are at least 1µm in average diameter size are in fluid communication with at least one adjoining cell.

In addition to being open-celled, in certain embodiments foams are sufficiently hydrophilic to permit the foam to absorb aqueous fluids, for example the internal surfaces of a foam may be rendered hydrophilic by residual hydrophilizing surfactants or salts left in the foam following polymerization, by selected post-polymerization foam treatment procedures (as described hereafter), or combinations of both.

In certain embodiments, for example when used in certain absorbent articles, an open-cell foam may be flexible and exhibit an appropriate glass transition temperature (Tg). The Tg represents the midpoint of the transition between the glassy and rubbery states of the polymer.

In certain embodiments, the Tg of this region will be less than about 200° C for foams used at about ambient temperature conditions, in certain other embodiments less than about 90° C. The Tg may be less than 50° C.

The open-cell foam pieces may be distributed in any suitable manner throughout the heterogeneous mass. In an embodiment, the open-cell foam pieces may be profiled along the vertical axis such that smaller pieces are located above larger pieces. Alternatively, the pieces may be profiled such that smaller pieces are below larger pieces. In another embodiment, the open-cell pieces may be profiled along a vertical axis such that they alternate in size along the axis.

In an embodiment the open-cell foam pieces may be profiled along any one of the longitudinal, lateral, or vertical axis based on one or more characteristics of the open-cell foam pieces. Characteristics by which the open-cell foam pieces may be profiled within the heterogeneous mass may include, for example, absorbency, density, cell size, and combinations thereof.

In an embodiment, the open-cell foam pieces may be profiled along any one of the longitudinal, lateral, or vertical axis based on the composition of the open-cell foam. The open-cell foam pieces may have one composition exhibiting desirable characteristics in the front of the heterogeneous mass and a different composition in the back of the heterogeneous mass designed to exhibit different characteristics. The profiling of the open-cell foam pieces may be either symmetric or asymmetric about any of the prior mentioned axes or orientations.

The open-cell foam pieces may be distributed along the longitudinal and lateral axis of the heterogeneous mass in any suitable form. In an embodiment, the open-cell foam pieces may be distributed in a manner that forms a design or shape when viewed from a top planar view. The open-cell foam pieces may be distributed in a manner that forms stripes, ellipticals, squares, or any other known shape or pattern.

In an embodiment, different types of foams may be used in one heterogeneous mass. For example, some of the foam pieces may be polymerized HIPE while other pieces may be made from polyurethane. The pieces may be located at specific locations within the mass based on their properties to optimize the performance of the heterogeneous mass.

In an embodiment, the open-celled foam is a thermoset polymeric foam made from the polymerization of a High Internal Phase Emulsion (HIPE), also referred to as a polyHIPE. To form a HIPE, an aqueous phase and an oil phase are combined in a ratio between about 8:1 and 140:1. In certain embodiments, the aqueous phase to oil phase ratio is between about 10:1 and about 75:1, and in certain other embodiments the aqueous phase to oil phase ratio is between about 13:1 and about 65:1. This is termed the "water-to-oil" or W:O ratio and can be used to determine the density of the resulting polyHIPE foam. As discussed, the oil phase may contain one or more of monomers, co-monomers, photo-initiators, cross-linkers, and emulsifiers, as well as optional components. The water phase will contain water and in certain embodiments one or more components such as electrolyte, initiator, or optional components.

The open-cell foam can be formed from the combined aqueous and oil phases by subjecting these combined phases to shear agitation in a mixing chamber or mixing zone. The combined aqueous and oil phases are subjected to shear agitation to produce a stable HIPE having aqueous droplets of the desired size. An initiator may be present in the aqueous phase, or an initiator may be introduced during the foam making process, and in certain embodiments, after the HIPE has been formed. The emulsion making process produces a HIPE where the aqueous phase droplets are dispersed to such an extent that the resulting HIPE foam will have the desired structural characteristics. Emulsification of the aqueous and oil phase combination in the mixing zone may involve the use of a mixing or agitation device such as an impeller, by passing the combined aqueous and oil phases through a series of static mixers at a rate necessary to impart the requisite shear, or combinations of both. Once formed, the HIPE can then be withdrawn or pumped from the mixing zone. One method for forming HIPEs using a continuous process is described in U.S. Pat. No. 5,149,720 (DesMarais et al), issued Sep. 22, 1992; U.S. Pat. No. 5,827,909 (DesMarais) issued Oct. 27, 1998; and U.S. Pat. No. 6,369,121 (Catalfamo et al.) issued Apr. 9, 2002.

The emulsion can be withdrawn or pumped from the mixing zone and impregnated into or onto a mass prior to being fully polymerized. Once fully polymerized, the foam pieces and the elements are intertwined such that discrete foam pieces are bisected by the elements comprising the mass and such that parts of discrete foam pieces enrobe portions of one or more of the elements comprising the heterogeneous mass.

Following polymerization, the resulting foam pieces are saturated with aqueous phase that needs to be removed to obtain substantially dry foam pieces. In certain embodiments, foam pieces can be squeezed free of most of the aqueous phase by using compression, for example by running the heterogeneous mass comprising the foam pieces through one or more pairs of nip rollers. The nip rollers can be positioned such that they squeeze the aqueous phase out of the foam pieces. The nip rollers can be porous and have a vacuum applied from the inside such that they assist in drawing aqueous phase out of the foam pieces. In certain embodiments, nip rollers can be positioned in pairs, such that a first nip roller is located above a liquid permeable belt, such as a belt having pores or composed of a mesh-like material and a second opposing nip roller facing the first nip roller and located below the liquid permeable belt. One of the pair, for example the first nip roller can be pressurized while the other, for example the second nip roller, can be evacuated, so as to both blow and draw the aqueous phase out the of the foam. The nip rollers may also be heated to assist in removing the aqueous phase. In certain embodiments, nip rollers are only applied to non-rigid foams, that is, foams whose walls would not be destroyed by compressing the foam pieces.

In certain embodiments, in place of or in combination with nip rollers, the aqueous phase may be removed by sending the foam pieces through a drying zone where it is heated, exposed to a vacuum, or a combination of heat and vacuum exposure. Heat can be applied, for example, by running the foam though a forced air oven, IR oven, microwave oven or radiowave oven. The extent to which a foam is dried depends on the application. In certain embodiments, greater than 50% of the aqueous phase is removed. In certain other embodiments greater than 90%, and in still other embodiments greater than 95% of the aqueous phase is removed during the drying process.

In an embodiment, open-cell foam is produced from the polymerization of the monomers having a continuous oil phase of a High Internal Phase Emulsion (HIPE). The HIPE may have two phases. One phase is a continuous oil phase having monomers that are polymerized to form a HIPE foam and an emulsifier to help stabilize the HIPE. The oil phase may also include one or more photo-initiators. The monomer component may be present in an amount of from about 80% to about 99%, and in certain embodiments from about 85% to about 95% by weight of the oil phase. The emulsifier component, which is soluble in the oil phase and suitable for forming a stable water-in-oil emulsion may be present in the oil phase in an amount of from about 1% to about 20% by weight of the oil phase. The emulsion may be formed at an emulsification temperature of from about 10° C to about 130° C and in certain embodiments from about 50° C to about 100° C.

In general, the monomers will include from about 20% to about 97% by weight of the oil phase at least one substantially water-insoluble monofunctional alkyl acrylate or alkyl methacrylate. For example, monomers of this type may include C₄-C₁₈ alkyl acrylates and C₂-C₁₈ methacrylates, such as ethylhexyl acrylate, butyl acrylate, hexyl acrylate, octyl acrylate, nonyl acrylate, decyl acrylate, isodecyl acrylate, tetradecyl acrylate, benzyl acrylate, nonyl phenyl acrylate, hexyl methacrylate, 2-ethylhexyl methacrylate, octyl methacrylate, nonyl methacrylate, decyl methacrylate, isodecyl methacrylate, dodecyl methacrylate, tetradecyl methacrylate, and octadecyl methacrylate.

The oil phase may also have from about 2% to about 40%, and in certain embodiments from about 10% to about 30%, by weight of the oil phase, a substantially water-insoluble, polyfunctional crosslinking alkyl acrylate or methacrylate. This crosslinking co-monomer, or cross-linker, is added to confer strength and resilience to the resulting HIPE foam. Examples of crosslinking monomers of this type may have monomers containing two or more activated acrylate, methacrylate groups, or combinations thereof. Nonlimiting examples of this group include 1,6-hexanedioldiacrylate, 1,4-butanedioldimethacrylate, trimethylolpropane triacrylate, trimethylolpropane trimethacrylate, 1,12-dodecyldimethacrylate, 1,14-tetradecanedioldimethacrylate, ethylene glycol dimethacrylate, neopentyl glycol diacrylate (2,2-dimethylpropanediol diacrylate), hexanediol acrylate methacrylate, glucose pentaacrylate, sorbitan pentaacrylate, and the like. Other examples of cross-linkers contain a mixture of acrylate and methacrylate moieties, such as ethylene glycol acrylate-methacrylate and neopentyl glycol acrylate-methacrylate. The ratio of methacrylate:acrylate group in the mixed cross-linker may be varied from 50:50 to any other ratio as needed.

Any third substantially water-insoluble co-monomer may be added to the oil phase in weight percentages of from about 0% to about 15% by weight of the oil phase, in certain embodiments from about 2% to about 8%, to modify properties of the HIPE foams. In certain embodiments, "toughening" monomers may be desired which impart toughness to the resulting HIPE foam. These include monomers such as styrene, vinyl chloride, vinylidene chloride, isoprene, and chloroprene. Without being bound by theory, it is believed that such monomers aid in stabilizing the HIPE during polymerization (also known as "curing") to provide a more homogeneous and better formed HIPE foam which results in better toughness, tensile strength, abrasion resistance, and the like. Monomers may also be added to confer flame retardancy as disclosed in U.S. Pat. No. 6,160,028 (Dyer) issued Dec. 12, 2000. Monomers may be added to confer color, for example vinyl ferrocene, fluorescent properties, radiation resistance, opacity to radiation, for example lead tetraacrylate, to disperse charge, to reflect incident infrared light, to absorb radio waves, to form a wettable surface on the HIPE foam struts, or for any other desired property in a HIPE foam. In some cases, these additional monomers may slow the overall process of conversion of HIPE to HIPE foam, the tradeoff being necessary if the desired property is to be conferred. Thus, such monomers can be used to slow down the polymerization rate of a HIPE. Examples of monomers of this type can have styrene and vinyl chloride.

The oil phase may further contain an emulsifier used for stabilizing the HIPE. Emulsifiers used in a HIPE can include: (a) sorbitan monoesters of branched C₁₆-C₂₄ fatty acids; linear unsaturated C₁₆-C₂₂ fatty acids; and linear saturated C₁₂-C₁₄ fatty acids, such as sorbitan monooleate, sorbitan monomyristate, and sorbitan monoesters, sorbitan monolaurate diglycerol monooleate (DGMO), polyglycerol monoisostearate (PGMIS), and polyglycerol monomyristate (PGMM); (b) polyglycerol monoesters of -branched C₁₆-C₂₄ fatty acids, linear unsaturated C₁₆-C₂₂ fatty acids, or linear saturated C₁₂-C₁₄ fatty acids, such as diglycerol monooleate (for example diglycerol monoesters of C18:1 fatty acids), diglycerol monomyristate, diglycerol monoisostearate, and diglycerol monoesters; (c) diglycerol monoaliphatic ethers of -branched C₁₆-C₂₄ alcohols, linear unsaturated C₁₆-C₂₂ alcohols, and linear saturated C₁₂-C₁₄ alcohols, and mixtures of these emulsifiers. See U.S. Pat. No. 5,287,207 (Dyer et al.), issued Feb. 7, 1995 and U.S. Pat. No. 5,500,451 (Goldman et al.) issued Mar. 19, 1996. Another emulsifier that may be used is polyglycerol succinate (PGS), which is formed from an alkyl succinate, glycerol, and triglycerol.

Such emulsifiers, and combinations thereof, may be added to the oil phase so that they can have between about 1% and about 20%, in certain embodiments from about 2% to about 15%, and in certain other embodiments from about 3% to about 12% by weight of the oil phase. In certain embodiments, co-emulsifiers may also be used to provide additional control of cell size, cell size distribution, and emulsion stability, particularly at higher temperatures, for example greater than about 65° C. Examples of co-emulsifiers include phosphatidyl cholines and phosphatidyl choline-containing compositions, aliphatic betaines, long chain C₁₂-C₂₂ dialiphatic quaternary ammonium salts, short chain C₁-C₄ dialiphatic quaternary ammonium salts, long chain C₁₂-C₂₂ dialkoyl(alkenoyl)-2-hydroxyethyl, short chain C₁-C₄ dialiphatic quaternary ammonium salts, long chain C₁₂-C₂₂ dialiphatic imidazolinium quaternary ammonium salts, short chain C₁-C₄ dialiphatic imidazolinium quaternary ammonium salts, long chain C₁₂-C₂₂ monoaliphatic benzyl quaternary ammonium salts, long chain C₁₂-C₂₂ dialkoyl(alkenoyl)-2-aminoethyl, short chain C₁-C₄ monoaliphatic benzyl quaternary ammonium salts, short chain C₁-C₄ monohydroxyaliphatic quaternary ammonium salts. In certain embodiments, ditallow dimethyl ammonium methyl sulfate (DTDMAMS) may be used as a co-emulsifier.

The oil phase may comprise a photo-initiator at between about 0.05% and about 10%, and in certain embodiments between about 0.2% and about 10% by weight of the oil phase. Lower amounts of photo-initiator allow light to better penetrate the HIPE foam, which can provide for polymerization deeper into the HIPE foam. However, if polymerization is done in an oxygen-containing environment, there should be enough photo-initiator to initiate the polymerization and overcome oxygen inhibition. Photo-initiators can respond rapidly and efficiently to a light source with the production of radicals, cations, and other species that are capable of initiating a polymerization reaction. The photo-initiators used in the present invention may absorb UV light at wavelengths of about 200 nanometers (nm) to about 800 nm, in certain embodiments about 200 nm to about 350 nm. If the photo-initiator is in the oil phase, suitable types of oil-soluble photo-initiators include benzyl ketals, α-hydroxyalkyl phenones, α-amino alkyl phenones, and acylphospine oxides. Examples of photo-initiators include 2,4,6-[trimethylbenzoyldiphosphine] oxide in combination with 2-hydroxy-2-methyl-1-phenylpropan-1-one (50:50 blend of the two is sold by Ciba Speciality Chemicals, Ludwigshafen, Germany as DAROCUR^{®} 4265); benzyl dimethyl ketal (sold by Ciba Geigy as IRGACURE 651); α-,α-dimethoxy-α-hydroxy acetophenone (sold by Ciba Speciality Chemicals as DAROCUR^{®} 1173); 2-methyl-1-[4-(methyl thio) phenyl]-2-morpholino-propan-1-one (sold by Ciba Speciality Chemicals as IRGACURE^{®} 907); 1-hydroxycyclohexyl-phenyl ketone (sold by Ciba Speciality Chemicals as IRGACURE^{®} 184); bis(2,4,6-trimethylbenzoyl)-phenylphosphineoxide (sold by Ciba Speciality Chemicals as IRGACURE 819); diethoxyacetophenone, and 4-(2-hydroxyethoxy)phenyl-(2-hydroxy-2-methylpropyl) ketone (sold by Ciba Speciality Chemicals as IRGACURE^{®} 2959); and Oligo [2-hydroxy-2-methyl-1-[4-(1-methylvinyl) phenyl]propanone] (sold by Lamberti spa, Gallarate, Italy as ESACURE^{®} KIP EM.

The dispersed aqueous phase of a HIPE can have water, and may also have one or more components, such as initiator, photo-initiator, or electrolyte, wherein in certain embodiments, the one or more components are at least partially water soluble.

One component of the aqueous phase may be a water-soluble electrolyte. The water phase may contain from about 0.2% to about 40%, in certain embodiments from about 2% to about 20%, by weight of the aqueous phase of a water-soluble electrolyte. The electrolyte minimizes the tendency of monomers, co-monomers, and cross-linkers that are primarily oil soluble to also dissolve in the aqueous phase. Examples of electrolytes include chlorides or sulfates of alkaline earth metals such as calcium or magnesium and chlorides or sulfates of alkali earth metals such as sodium. Such electrolyte can include a buffering agent for the control of pH during the polymerization, including such inorganic counter-ions as phosphate, borate, and carbonate, and mixtures thereof. Water soluble monomers may also be used in the aqueous phase, examples being acrylic acid and vinyl acetate.

Another component that may be present in the aqueous phase is a water-soluble free-radical initiator. The initiator can be present at up to about 20 mole percent based on the total moles of polymerizable monomers present in the oil phase. In certain embodiments, the initiator is present in an amount of from about 0.001 to about 10 mole percent based on the total moles of polymerizable monomers in the oil phase. Suitable initiators include ammonium persulfate, sodium persulfate, potassium persulfate, 2,2'-azobis(N,N'-dimethyleneisobutyramidine)dihydrochloride, and other suitable azo initiators. In certain embodiments, to reduce the potential for premature polymerization which may clog the emulsification system, addition of the initiator to the monomer phase may be just after or near the end of emulsification.

Photo-initiators present in the aqueous phase may be at least partially water soluble and can have between about 0.05% and about 10%, and in certain embodiments between about 0.2% and about 10% by weight of the aqueous phase. Lower amounts of photo-initiator allow light to better penetrate the HIPE foam, which can provide for polymerization deeper into the HIPE foam. However, if polymerization is done in an oxygen-containing environment, there should be enough photo-initiator to initiate the polymerization and overcome oxygen inhibition. Photo-initiators can respond rapidly and efficiently to a light source with the production of radicals, cations, and other species that are capable of initiating a polymerization reaction. The photo-initiators used in the present invention may absorb UV light at wavelengths of from about 200 nanometers (nm) to about 800 nm, in certain embodiments from about 200 nm to about 350 nm, and in certain embodiments from about 350 nm to about 450 nm. If the photo-initiator is in the aqueous phase, suitable types of water-soluble photo-initiators include benzophenones, benzils, and thioxanthones. Examples of photo-initiators include 2,2'-Azobis[2-(2-imidazolin-2-yl)propane]dihydrochloride; 2,2'-Azobis[2-(2-imidazolin-2-yl)propane]disulfate dehydrate; 2,2'-Azobis(1-imino-1-pyrrolidino-2-ethylpropane)dihydrochloride; 2,2'-Azobis[2-methyl-N-(2-hydroxyethyl)propionamide]; 2,2'-Azobis(2-methylpropionamidine)dihydrochloride; 2,2'-dicarboxymethoxydibenzalacetone, 4,4'-dicarboxymethoxydibenzalacetone, 4,4'-dicarboxymethoxydibenzalcyclohexanone,4-dimethylamino-4'-carboxymethoxydibenzalacetone; and 4,4'-disulphoxymethoxydibenzalacetone. Other suitable photo-initiators that can be used in the present invention are listed in U.S. Pat. No. 4,824,765 (Sperry et al.) issued Apr. 25, 1989.

In addition to the previously described components other components may be included in either the aqueous or oil phase of a HIPE. Examples include antioxidants, for example hindered phenolics, hindered amine light stabilizers; plasticizers, for example dioctyl phthalate, dinonyl sebacate; flame retardants, for example halogenated hydrocarbons, phosphates, borates, inorganic salts such as antimony trioxide or ammonium phosphate or magnesium hydroxide; dyes and pigments; fluorescers; filler pieces, for example starch, titanium dioxide, carbon black, or calcium carbonate; fibers; chain transfer agents; odor absorbers, for example activated carbon particulates; dissolved polymers; dissolved oligomers; and the like.

The heterogeneous mass comprises a HIPE foam (25) intermixed enrobeable elements in the form of a nonwoven web.and discrete pieces of foam.

The enrobeable elements may also be fibers such as, for example, synthetic fibers, thermoplastic particulates or fibers, tricomponent fibers, and bicomponent fibers such as, for example, sheath/core fibers having the following polymer combinations: polyethylene/polypropylene, polyethylvinyl acetate/polypropylene, polyethylene/polyester, polypropylene/polyester, copolyester/polyester, and the like. The enrobeable elements may be any combination of the materials listed above and/or a plurality of the materials listed above, alone or in combination.

The enrobeable elements may be hydrophobic or hydrophilic. In an embodiment, the enrobeable elements may be treated to be made hydrophobic. In an embodiment, the enrobeable elements may be treated to become hydrophilic.

The constituent fibers of the heterogeneous mass can be comprised of polymers such as polyethylene, polypropylene, polyester, and blends thereof. The fibers can be spunbond fibers. The fibers can be meltblown fibers. The fibers can comprise cellulose, rayon, cotton, or other natural materials or blends of polymer and natural materials. The fibers can also comprise a super absorbent material such as polyacrylate or any combination of suitable materials. The fibers can be monocomponent, bicomponent, and/or biconstituent, non-round (e.g., capillary channel fibers), and can have major cross-sectional dimensions (e.g., diameter for round fibers) ranging from 0.1-500 microns. The constituent fibers of the nonwoven precursor web may also be a mixture of different fiber types, differing in such features as chemistry (e.g. polyethylene and polypropylene), components (mono- and bi-), denier (micro denier and >20 denier), shape (i.e. capillary and round) and the like. The constituent fibers can range from about 0.1 denier to about 100 denier.

In one aspect, known absorbent web materials in an as-made can be considered as being homogeneous throughout. Being homogeneous, the fluid handling properties of the absorbent web material are not location dependent, but are substantially uniform at any area of the web. Homogeneity can be characterized by density, basis weight, for example, such that the density or basis weight of any particular part of the web is substantially the same as an average density or basis weight for the web. By the apparatus and method of the present invention, homogeneous fibrous absorbent web materials are modified such that they are no longer homogeneous, but are heterogeneous, such that the fluid handling properties of the web material are location dependent. Therefore, for the heterogeneous absorbent materials of the present invention, at discrete locations the density or basis weight of the web may be substantially different than the average density or basis weight for the web. The heterogeneous nature of the absorbent web of the present invention permits the negative aspects of either of permeability or capillarity to be minimized by rendering discrete portions highly permeable and other discrete portions to have high capillarity. Likewise, the tradeoff between permeability and capillarity is managed such that delivering relatively higher permeability can be accomplished without a decrease in capillarity.

In an embodiment, the heterogeneous mass may also include superabsorbent material that imbibe fluids and form hydrogels. These materials are typically capable of absorbing large quantities of body fluids and retaining them under moderate pressures. The heterogeneous mass can include such materials dispersed in a suitable carrier such as cellulose fibers in the form of fluff or stiffened fibers.

In an embodiment, the heterogeneous mass may include thermoplastic particulates or fibers. The materials, and in particular thermoplastic fibers, can be made from a variety of thermoplastic polymers including polyolefins such as polyethylene (e.g., PULPEX.RTM.) and polypropylene, polyesters, copolyesters, and copolymers of any of the foregoing.

Depending upon the desired characteristics, suitable thermoplastic materials include hydrophobic fibers that have been made hydrophilic, such as surfactant-treated or silica-treated thermoplastic fibers derived from, for example, polyolefins such as polyethylene or polypropylene, polyacrylics, polyamides, polystyrenes, and the like. The surface of the hydrophobic thermoplastic fiber can be rendered hydrophilic by treatment with a surfactant, such as a nonionic or anionic surfactant, e.g., by spraying the fiber with a surfactant, by dipping the fiber into a surfactant or by including the surfactant as part of the polymer melt in producing the thermoplastic fiber. Upon melting and resolidification, the surfactant will tend to remain at the surfaces of the thermoplastic fiber. Suitable surfactants include nonionic surfactants such as Brij 76 manufactured by ICI Americas, Inc. of Wilmington, Del., and various surfactants sold under the Pegosperse.RTM. trademark by Glyco Chemical, Inc. of Greenwich, Conn. Besides nonionic surfactants, anionic surfactants can also be used. These surfactants can be applied to the thermoplastic fibers at levels of, for example, from about 0.2 to about 1 g. per sq. of centimeter of thermoplastic fiber.

Suitable thermoplastic fibers can be made from a single polymer (monocomponent fibers), or can be made from more than one polymer (e.g., bicomponent fibers). The polymer comprising the sheath often melts at a different, typically lower, temperature than the polymer comprising the core. As a result, these bicomponent fibers provide thermal bonding due to melting of the sheath polymer, while retaining the desirable strength characteristics of the core polymer.

Suitable bicomponent fibers for use in the present invention can include sheath/core fibers having the following polymer combinations: polyethylene/polypropylene, polyethylvinyl acetate/polypropylene, polyethylene/polyester, polypropylene/polyester, copolyester/polyester, and the like. Particularly suitable bicomponent thermoplastic fibers for use herein are those having a polypropylene or polyester core, and a lower melting copolyester, polyethylvinyl acetate or polyethylene sheath (e.g., DANAKLON.RTM., CELBOND.RTM. or CHISSO.RTM. bicomponent fibers). These bicomponent fibers can be concentric or eccentric. As used herein, the terms "concentric" and "eccentric" refer to whether the sheath has a thickness that is even, or uneven, through the cross-sectional area of the bicomponent fiber. Eccentric bicomponent fibers can be desirable in providing more compressive strength at lower fiber thicknesses. Suitable bicomponent fibers for use herein can be either uncrimped (i.e. unbent) or crimped (i.e. bent). Bicomponent fibers can be crimped by typical textile means such as, for example, a stuffer box method or the gear crimp method to achieve a predominantly two-dimensional or "flat" crimp.

The length of bicomponent fibers can vary depending upon the particular properties desired for the fibers and the web formation process. Typically, in an airlaid web, these thermoplastic fibers have a length from about 2mm to about 12mm long, preferably from about 2.5mm to about 7.5mm long, and most preferably from about 3.0mm to about 6.0mm long. The properties-of these thermoplastic fibers can also be adjusted by varying the diameter (caliper) of the fibers. The diameter of these thermoplastic fibers is typically defined in terms of either denier (grams per 9000 meters) or decitex (grams per 10,000 meters). Suitable bicomponent thermoplastic fibers as used in an airlaid making machine can have a decitex in the range from about 1.0 to about 20, preferably from about 1.4 to about 10, and most preferably from about 1.7 to about 7 decitex.

The compressive modulus of these thermoplastic materials, and especially that of the thermoplastic fibers, can also be important. The compressive modulus of thermoplastic fibers is affected not only by their length and diameter, but also by the composition and properties of the polymer or polymers from which they are made, the shape and configuration of the fibers (e.g., concentric or eccentric, crimped or uncrimped), and like factors. Differences in the compressive modulus of these thermoplastic fibers can be used to alter the properties, and especially the density characteristics, of the respective thermally bonded fibrous matrix.

The heterogeneous mass can also include synthetic fibers that typically do not function as binder fibers but alter the mechanical properties of the fibrous webs. Synthetic fibers include cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. These might include, for example, polyester fibers such as polyethylene terephthalate (e.g., DACRON.RTM. and KODEL.RTM.), high melting crimped polyester fibers (e.g., KODEL.RTM. 431 made by Eastman Chemical Co.) hydrophilic nylon (HYDROFIL.RTM.), and the like. Suitable fibers can also hydrophilized hydrophobic fibers, such as surfactant-treated or silica-treated thermoplastic fibers derived from, for example, polyolefins such as polyethylene or polypropylene, polyacrylics, polyamides, polystyrenes, polyurethanes and the like. In the case of nonbonding thermoplastic fibers, their length can vary depending upon the particular properties desired for these fibers. Typically they have a length from about 0.3 to 7.5 cm, preferably from about 0.9 to about 1.5 cm. Suitable nonbonding thermoplastic fibers can have a decitex in the range of about 1.5 to about 35 decitex, more preferably from about 14 to about 20 decitex.

However structured, the total absorbent capacity of the heterogeneous mass containing foam pieces should be compatible with the design loading and the intended use of the mass. For example, when used in an absorbent article, the size and absorbent capacity of the heterogeneous mass may be varied to accommodate different uses such as incontinence pads, pantiliners, regular sanitary napkins, or overnight sanitary napkins.

The heterogeneous mass can also include other optional components sometimes used in absorbent webs. For example, a reinforcing scrim can be positioned within the respective layers, or between the respective layers, of the heterogeneous mass.

The heterogeneous mass comprising open-cell foam pieces produced from the present invention may be used as an absorbent core or a portion of an absorbent core in absorbent articles, such as feminine hygiene articles, for example pads, pantiliners, and tampons; disposable diapers; incontinence articles, for example pads, adult diapers; homecare articles, for example wipes, pads, towels; and beauty care articles, for example pads, wipes, and skin care articles, such as used for pore cleaning.

The absorbent core structure is used as an absorbent core for an absorbent article. In such an embodiment, the absorbent core can be relatively thin, less than about 5 mm in thickness, or less than about 3 mm, or less than about 1 mm in thickness. Cores having a thickness of greater than 5 mm are also contemplated herein. Thickness can be determined by measuring the thickness at the midpoint along the longitudinal centerline of the pad by any means known in the art for doing while under a uniform pressure of 0.25 psi. The absorbent core can comprise absorbent gelling materials (AGM), including AGM fibers, as is known in the art.

The heterogeneous mass layer may be formed or cut to a shape, the outer edges of which define a periphery.

The open-cell foam pieces are in the form of stripes. The stripes may be formed during the formation of the heterogeneous mass or by formation means after polymerization. The stripes may run along the longitudinal length of the heterogeneous mass layer, along the lateral length of the heterogeneous mass layer, or a combination of both the longitudinal length and the lateral length. The stripes may run along a diagonal to either the longitudinal length or the lateral length of the heterogeneous mass layer. The stripes are separated by canals.

Formation means known for deforming a generally planar fibrous web into a three-dimensional structure are utilized in the present invention to modify as-made absorbent materials into absorbent materials having relatively higher permeability without a significant corresponding decrease in capillary pressure. Formation means may comprise a pair of inter-meshing rolls, typically steel rolls having inter-engaging ridges or teeth and grooves. However, it is contemplated that other means for achieving formation can be utilized, such as the deforming roller and cord arrangement disclosed in US 2005/0140057 published June 30, 2005. Therefore, all disclosure of a pair of rolls herein is considered equivalent to a roll and cord, and a claimed arrangement reciting two inter-meshing rolls is considered equivalent to an inter-meshing roll and cord where a cord functions as the ridges of a mating inter-engaging roll. In one embodiment, the pair of intermeshing rolls of the instant invention can be considered as equivalent to a roll and an inter-meshing element, wherein the inter-meshing element can be another roll, a cord, a plurality of cords, a belt, a pliable web, or straps. Likewise, other known formation technologies, such as creping, necking/consolidation, corrugating, embossing, button break, hot pin punching, and the like are believed to be able to produce absorbent materials having some degree of relatively higher permeability without a significant corresponding decrease in capillary pressure. Formation means utilizing rolls include "ring rolling", a "SELF" or "SELF'ing" process, in which SELF stands for Structural Elastic Like Film, as "micro-SELF", and "rotary knife aperturing" (RKA); as described in US Patent No. 7,935,207 Zhao et al., granted May 3, 2011.

The absorbent core structure has an absorbent layer that comprises superabsorbent particles. The superabsorbent particles may be on a substrate or within a nonwoven layer. The absorbent layer may additionally comprise a thermoplastic. In an embodiment, the absorbent core layer may comprise of any layer or combination of layers as described in US 8,263,820; US 8,124,827; US patent publication no. 2010-0228209 A1; or US patent publication no. 2010-0262104 A1.

The substrate of the absorbent layer can advantageously comprise a fibrous material substantially free of cellulose fibers. By saying that a layer of the absorbent core is "substantially free" of cellulose fibers, it is meant in the context of the present invention that the layer should not comprise any significant amount of cellulose fibers within its inner structure. While cellulose fibers which can be present at an outer surface of the specified layer, for example at the interface between the specified layer and an adjacent one, which could be for example an outer layer wrapping the core, in some cases can accidentally and slightly penetrate the structure of the specified layer, such shall not be considered significant. Significant amount can correspond to less than 10% by weight, less than 5% by weight, less than 3% by weight, or less than 1% by weight, based on the dry weight of the specified layer of the absorbent core. The substrate layer 100 can also have a basis weight from 25 g/m² to 120 g/m², or from 35 g/m² to 90 g/m².

The thermoplastic material may comprise, in its entirety, a single thermoplastic polymer or a blend of thermoplastic polymers, having a softening point, as determined by the ASTM Method D-36-95 "Ring and Ball", in the range between 50°C and 300°C, or alternatively the thermoplastic composition may be a hot melt adhesive comprising at least one thermoplastic polymer in combination with other thermoplastic diluents such as tackifying resins, plasticizers and additives such as antioxidants.

The thermoplastic polymer can have typically a molecular weight (Mw) of more than 10,000 and a glass transition temperature (Tg) usually below room temperature. Typical concentrations of the polymer in a hot melt are in the range of 20 - 40 % by weight. A wide variety of thermoplastic polymers can be suitable for use in the present invention. Such thermoplastic polymers can be typically water insensitive. Exemplary polymers can be (styrenic) block copolymers including A-B-A triblock structures, A-B diblock structures and (A-B)n radial block copolymer structures wherein the A blocks can be non-elastomeric polymer blocks, typically comprising polystyrene, and the B blocks can be unsaturated conjugated diene or (partly) hydrogenated versions of such. The B block can be typically isoprene, butadiene, ethylene/butylene (hydrogenated butadiene), ethylene/propylene (hydrogenated isoprene), and mixtures thereof.

Other suitable thermoplastic polymers that may be employed are metallocene polyolefins, which are ethylene polymers prepared using single-site or metallocene catalysts. Therein, at least one co-monomer can be polymerized with ethylene to make a copolymer, terpolymer or higher order polymer. Also applicable can be amorphous polyolefins or amorphous polyalphaolefins (APAO) which are homopolymers, copolymers or terpolymers of C2 to C8 alphaolefins.

The resin can typically have a Mw below 5,000 and a Tg usually above room temperature, typical concentrations of the resin in a hot melt can be in the range of 30 - 60 %. The plasticizer has a low Mw of typically less than 1,000 and a Tg below room temperature, a typical concentration is 0 -15%.

The thermoplastic material, typically a hotmelt adhesive, can be present in the form of fibers throughout the core, being provided with known means, i.e. the adhesive can be fiberized. Typically, the fibers can have an average thickness of 1 - 100 micrometer and an average length of 5 mm to 50 cm. In particular the layer of thermoplastic material, typically e.g. a hot melt adhesive, can be provided such as to comprise a net-like structure.

To improve the adhesiveness of the thermoplastic material to the substrate layer or to any other layer, in particular any other non-woven layer, such layers may be pre-treated with an auxiliary adhesive.

An absorbent core layer may have absorbent polymer material. Without wishing to be bound by theory it is believed that such material, even in the swollen state, i.e. when liquid has been absorbed, does not substantially obstruct the liquid flow throughout the material, particularly when further the permeability of said material, as expressed by the saline flow conductivity of the absorbent polymer material, is greater than 10, 20, 30 or 40 SFC- units, where 1 SFC unit is 1 x 10⁻⁷ (cm³ x s) / g. Saline flow conductivity is a parameter well recognized in the art and is to be measured in accordance with the test disclosed in EP 752 892 B.

This layer of absorbent polymer material can be typically a non-uniform layer, and comprises a first surface and a second surface, wherein by "non-uniform" it is meant that the absorbent polymer material is distributed over a substrate with non-uniform basis weight. Conversely, the second surface of the non-uniform layer of absorbent polymer material is in at least partial contact with the first surface of the substrate layer. According to an embodiment of the present invention, the non-uniform layer of absorbent polymer material can be a discontinuous layer that is a layer typically comprising openings, i.e. areas substantially free of absorbent polymer material, which in certain embodiments can be typically completely surrounded by areas comprising absorbent polymer material.

Suitable absorbent polymer materials for use in the invention can comprise a substantially water-insoluble, slightly crosslinked, partially neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Pat. No. 4,654,039 (Brandt et al), issued Mar. 31, 1987, and reissued as RE 32,649 on Apr. 19, 1988, both of which are incorporated by reference. Preferred monomers include acrylic acid, methacrylic acid, and 2-acrylamido-2-methyl propane sulfonic acid. Acrylic acid itself is especially preferred for preparation of the polymeric gelling material. The polymeric component formed from the unsaturated, acid-containing monomers can be grafted onto other types of polymer moieties such as starch or cellulose. Polyacrylate grafted starch materials of this type are especially preferred. Preferred polymeric absorbent gelling materials that can be prepared from conventional types of monomers include hydrolyzed acrylonitrile grafted starch, polyacrylate grafted starch, polyacrylates, maleic anhydride-based copolymers and combinations thereof.

According to an embodiment, an absorbent article can comprise a liquid pervious topsheet. The topsheet suitable for use herein can comprise wovens, non-wovens, and/or three-dimensional webs of a liquid impermeable polymeric film comprising liquid permeable apertures. The topsheet for use herein can be a single layer or may have a multiplicity of layers. For example, the wearer-facing and contacting surface can be provided by a film material having apertures which are provided to facilitate liquid transport from the wearer facing surface towards the absorbent structure. Such liquid permeable, apertured films are well known in the art. They provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

The absorbent layers may be combined using bonds, a bonding layer, adhesives, or combinations thereof. The absorbent core structure may be attached to the topsheet, the backsheet, or both the topsheet and backsheet using bonds, a bonding layer, adhesives, or combinations thereof. Adhesives may be placed in any suitable pattern, such as, for example, lines, spirals, points, circles, squares, or any other suitable pattern. Bonds may be placed in any suitable pattern, such as, for example, lines, spirals, points, circles, squares, or any other suitable pattern.

The absorbent layers may be combined using an intermediate layer between the two layers. The intermediate layer may comprise a tissue, a nonwoven, a film, or combinations thereof. The intermediate layer may have a permeability greater than the 200 Darcy.

The core structure is a two layer core structure. The upper layer is a heterogeneous mass comprising open-cell foam. The open-cell foam may comprise canals along the longitudinal length of the core. The lower layer comprises a substrate layer with superabsorbent polymer placed on top of the substrate. The substrate and superabsorbent polymer are coated by a thermoplastic. The two layer core structure may be combined with other layers provided that the additional layers are placed below the two layer core structure.

The canals within the upper layer of the two layer core structure may end before the edge of the core. The canals may between 0.1 inches and 3 inches from each end of the core, such as, for example, 0.2 inches, 0.25 inches, 0.3 inches, 0.35 inches, 0.4 inches, 0.45 inches, or 0.5 inches. Without being bound by theory, Applicants have found that the canals within the upper layer may carry the fluid away from the insult area making it accessible to portions of the lower core that would otherwise not see the fluid insult. The canals rapidly disperse fluid away from the loading or insult zone and utilize the void volume leading to faster acquisition times. At the same time, the canals provide high suction walls that provide active wicking of the fluids.

The canals may be spaced between 0.1 mm and 5 mm apart, such as for example, between 0.5 mm and 4 mm, or between 1 mm and 3 mm apart. In an embodiment, the canals are spaced such that they are parallel with each other and from 30% to 100%, or from 40% to 95%, or from 50% to 90%, or from 60% to 85% of the length of the longitudinal dimension, transverse dimension, lateral dimension, or a diagonal dimension of the heterogeneous mass top core structure. The canals may be continuous or discontinuous. The canals may parallel a longitudinal axis, a transverse axis, a lateral axis, or a diagonal axis of the heterogeneous mass top core structure. In an embodiment, the canals are in the form of sinusoidal waves versus straight lines. In an embodiment, the canals are in the form of any suitable geometric design such as, for example, spirals, swirls, lines, squares, waves, etc.

Without being bound by theory, Applicants have found that the two layer core structure described above allows for high capillarity suction while maintaining high permeability across an entire surface while maintaining a controlled ratio of permeability down through the canals relative to the capillarity of fluid through the canal walls. The ability to increase canal height and longitudinal flow rate creates a unique swelling phenomenon. Specifically, for a given insult amount, the overall swelling in the insult area associated with the superabsorbent containing layer is lower than ever before because the canals are so effective and transporting fluid away from the insult area. A series of parallel canals creates a structure that is unique relative to dimensional flexibility.

More specifically, the relative flexibility in the longitudinal direction (Flex_{L}) is significantly lower than the relative flexibility in the transverse direction of the pad (Flex_{T}), e.g. Flex_{L}/Flex_{T} > X, wherein X is in a new performance regime.

Further, the thin nature of the two layer absorbent core structure described above provides unique compressive and recovery energies relative to the product's capacity exhibiting a relationship between Capacity (Cap) and Flexibility (Flex) relative to thickness (T), e.g. ((Cap x Flex)/T) that is greater than for all prior products.

The absorbent core comprises of at least two layers. The top core layer comprises a heterogeneous mass containing a HIPE foam intermixed with a nonwoven web. The heterogeneous mass contains one or more stripes of high capillarity and one or more canals of high permeability. The canals of high permeability contain a percent of the HIPE foam compared to the amount of HIPE foam in the canals of high permeability, such as, for example, the canals of high permeability may have 50% or less of the amount of HIPE foam contained in the stripes of high capillarity, such as, for example, between 0% and 40% of the amount of HIPE foam in the stripes of high capillarity, between 0% and 30% of the amount of HIPE foam in the stripes of high capillarity, between 0% and 20% of the amount of HIPE foam in the stripes of high capillarity, and between 0% and 10% of the amount of HIPE foam in the stripes of high capillarity. The lower core level comprises an substrate plus super absorbent polymer layer containing greater than 50%, of a superabsorbent polymer and less than 30% of cellulose.

Without being bound by theory, Applicants have found that, when used as an absorbent core in an absorbent article, the canal walls of the heterogeneous mass comprising open-cell foam layer create a unique dynamic volume canal and fluid transport system based on liquid insults. More specifically, the canal walls vertically swell after liquid insults so that the canals are deeper and contain more volume capacity for subsequent insults. The canals do not significantly swell in the X-Y directions. The canals also maintain a relatively constant rate of fluid flow along the length of the canals due to the capillarity of the canal walls.

The two layer core structure allows for improved acquisition times for subsequent gushes.

**Table 1 shows the acquisition time for subsequent gushes of 20 ml at a rate of 10 ml/sec for multiple core structures.**

| Core Structure Examples | 1^{st} insult Acquisition Time | 2^{nd} Insult Acquisition Time | 3^{rd} Insult Acquisition Time | 4^{th} Insult Acquisition Time |
|---|---|---|---|---|
| Example A | 7 | 9 | 13 | 16 |
| Example B | 7 | 9 | 13 | 16 |
| Example C | 7 | 8 | 9 | 11 |
| Example D | 15 | 15 | 25 | 37 |
| Example E | 8 | 15 | 35 | 46 |

As shown in Table 1, the two layer core structure shown as Examples A to C exhibits a second gush acquisition time of less than 10 seconds, a third gush acquisition time of less than 15 seconds, and a fourth gush acquisition time of less than 20 seconds. Examples D and E are representative of absorbent core designs that do not incorporate the invention described in the application.

Examples A to C represent the same core system. Example B has an expanded upper layer compared to Example A. Example C has an expanded and stretched upper layer compared to Example A. Applicants have found that the appropriate level of expansion and stretch of the upper layer may further improve upon the acquisition times in seconds for subsequent second, third, and fourth gushes of fluid.

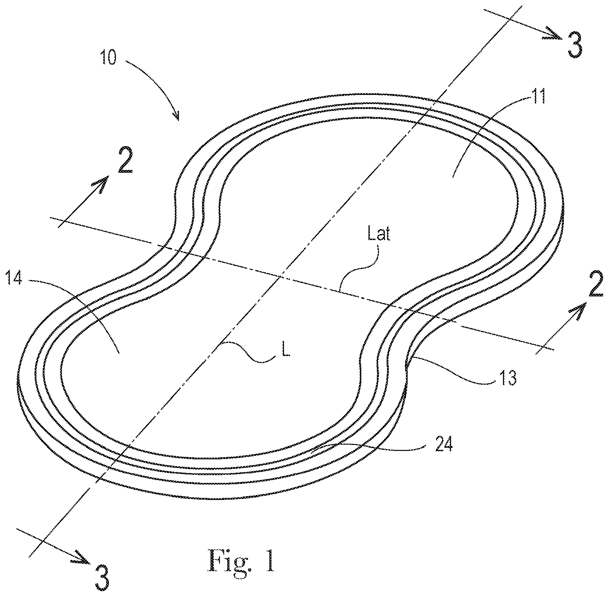

FIG. 1 a perspective view of one embodiment of a sanitary napkin. The illustrated sanitary napkin 10 has a body-facing upper side 11 that contacts the user's body during use. The opposite, garment-facing lower side 13 contacts the user's clothing during use.

A sanitary napkin 10 can have any shape known in the art for feminine hygiene articles, including the generally symmetric "hourglass" shape as shown in FIG. 1, as well as pear shapes, bicycle-seat shapes, trapezoidal shapes, wedge shapes or other shapes that have one end wider than the other. Sanitary napkins and pantyliners can also be provided with lateral extensions known in the art as "flaps" or "wings". Such extensions can serve a number of purposes, including, but not limited to, protecting the wearer's panties from soiling and keeping the sanitary napkin secured in place.

The upper side of a sanitary napkin generally has a liquid pervious topsheet 14. The lower side generally has a liquid impervious backsheet 16 that is joined with the topsheet 14 at the edges of the product. An absorbent core 18 is positioned between the topsheet 14 and the backsheet 16. A secondary topsheet may be provided at the top of the absorbent core 18, beneath the topsheet.

The topsheet 14, the backsheet 16, and the absorbent core 18 can be assembled in a variety of well- known configurations, including so called "tube" products or side flap products, such as, for example, configurations are described generally in U.S. Pat. No. 4,950,264, "Thin, Flexible Sanitary Napkin" issued to Osborn on Aug. 21, 1990, U.S. Pat. No. 4,425,130, "Compound Sanitary Napkin" issued to DesMarais on Jan. 10, 1984; U.S. Pat. No. 4,321,924, "Bordered Disposable Absorbent Article" issued to Ahr on Mar. 30, 1982; U.S. Pat. No. 4,589,876, and "Shaped Sanitary Napkin With Flaps" issued to Van Tilburg on Aug. 18, 1987. Each of these patents is incorporated herein by reference.

The backsheet 16 and the topsheet 14 can be secured together in a variety of ways. Adhesives manufactured by H. B. Fuller Company of St. Paul, Minn. under the designation HL-1258 or H-2031 have been found to be satisfactory. Alternatively, the topsheet 14 and the backsheet 16 can be joined to each other by heat bonding, pressure bonding, ultrasonic bonding, dynamic mechanical bonding, or a crimp seal. A fluid impermeable crimp seal 24 can resist lateral migration ("wicking") of fluid through the edges of the product, inhibiting side soiling of the wearer's undergarments.

As is typical for sanitary napkins and the like, the sanitary napkin 10 of the present invention can have panty-fastening adhesive disposed on the garment-facing side of backsheet 16. The panty-fastening adhesive can be any of known adhesives used in the art for this purpose, and can be covered prior to use by a release paper, as is well known in the art. If flaps or wings are present, panty fastening adhesive can be applied to the garment facing side so as to contact and adhere to the underside of the wearer's panties.

The backsheet may be used to prevent the fluids absorbed and contained in the absorbent structure from wetting materials that contact the absorbent article such as underpants, pants, pyjamas, undergarments, and shirts or jackets, thereby acting as a barrier to fluid transport. The backsheet according to an embodiment of the present invention can also allow the transfer of at least water vapour, or both water vapour and air through it.

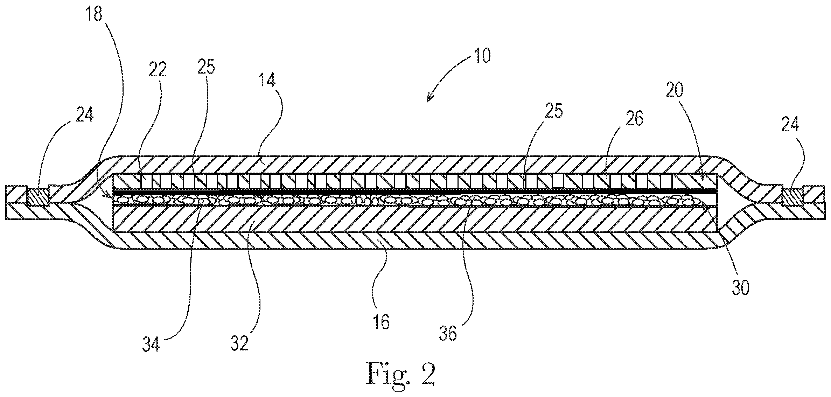

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin. FIG. 2 is a cross-sectional view of the sanitary napkin 10 of Fig. 1, taken through line 2-2. As shown in the figure, the absorbent core 18 structure comprises of an upper layer 20 and a lower layer 30. The upper layer 20 is a heterogeneous mass 22 comprising open-cell foam pieces 25. The open-cell foam pieces 25 are in the form of stripes 26 that run along the longitudinal length of the absorbent article 10. The absorbent foam pieces 25 are separated by canals 28. The absorbent core 18 structure lower layer 30 comprises a substrate 32 with superabsorbent polymer 34 on top of the substrate 32. The substrate 32 and polymer 34 are coated with a thermoplastic adhesive 36.

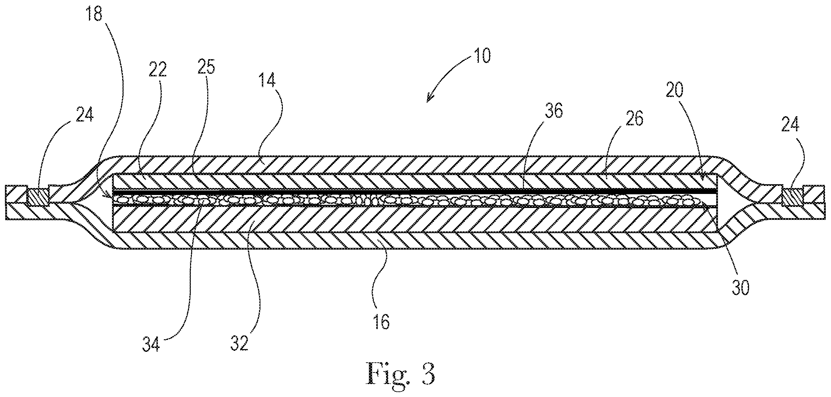

FIG. 3 is a cross-sectional view of the sanitary napkin 10 of Fig. 1, taken through line 3-3. As shown in the figure, the absorbent core 18 structure comprises of an upper layer 20 and a lower layer 30. The upper layer 20 is a heterogeneous mass 22 comprising open-cell foam pieces 25. The open-cell foam pieces 25 are in the form of stripes 26 that run along the longitudinal length of the absorbent article 10. The absorbent core 18 structure lower layer 30 comprises a substrate 32 with superabsorbent polymer 34 on top of the substrate 32. The substrate 32 and polymer 34 are coated with a thermoplastic adhesive 36.

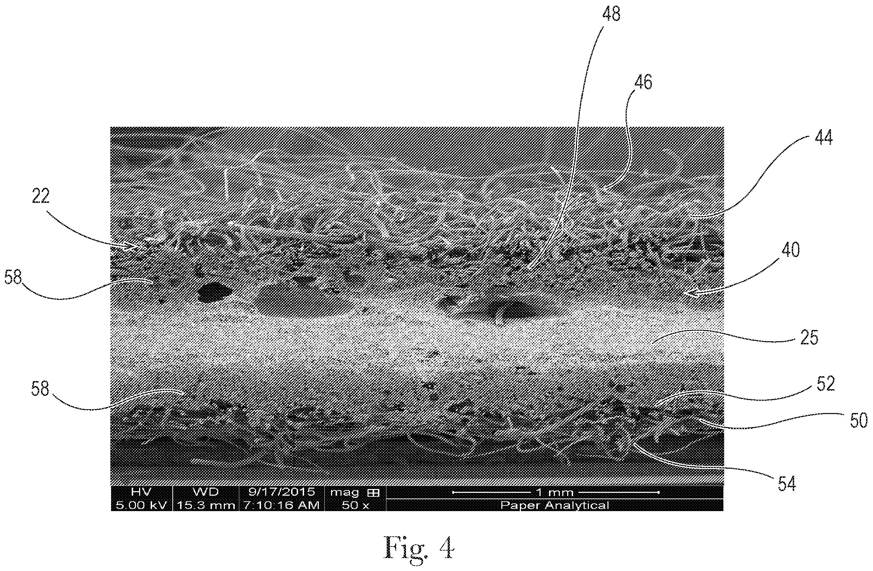

FIG. 4 is an SEM micrograph of a heterogeneous mass 22 prior to any formation means or forming of canals. As shown in FIG. 4, the absorbent stratum 40 is a heterogeneous mass 22 comprising a first planar nonwoven 44 having a first surface 46 and a second surface 48 and a second planar nonwoven 50 having a first surface 52 and a second surface 54. An open cell foam piece 25 enrobes a portion of the first planar nonwoven 44 and a portion of the second planar nonwoven 50. Specifically, the open cell foam piece 25 enrobes enrobeable elements 58 in both the second surface 48 of the first planar nonwoven 44 and the first surface 42 of the second planar nonwoven 50.

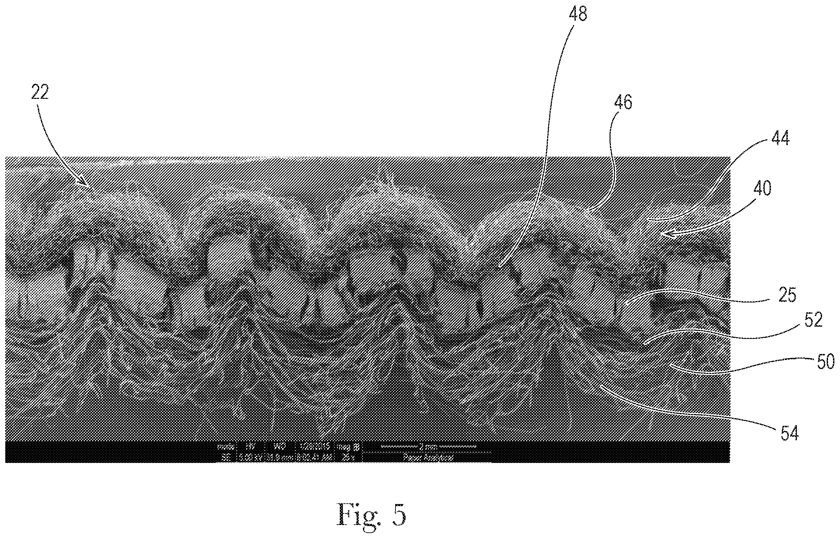

FIG. 5 is an SEM micrograph of a heterogeneous mass 22 after formation means or the forming of canals. As shown in FIG. 5, the absorbent stratum 40 is a heterogeneous mass 22 comprising a first planar nonwoven 44 having a first surface 46 and a second surface 48 and a second planar nonwoven 50 having a first surface 52 and a second surface 54. An open cell foam piece 25 enrobes a portion of the first planar nonwoven 44 and a portion of the second planar nonwoven 50. The planar nowovens are shown as wavy due to the impact of the formation means.

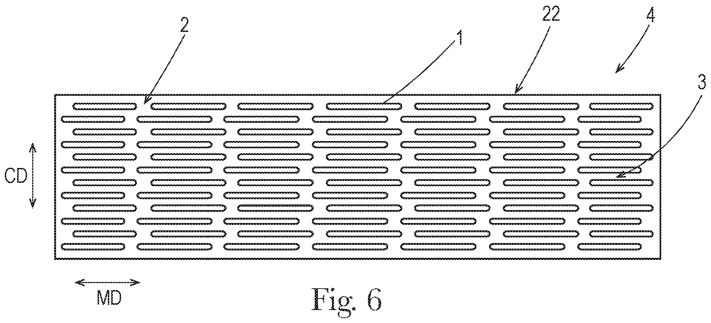

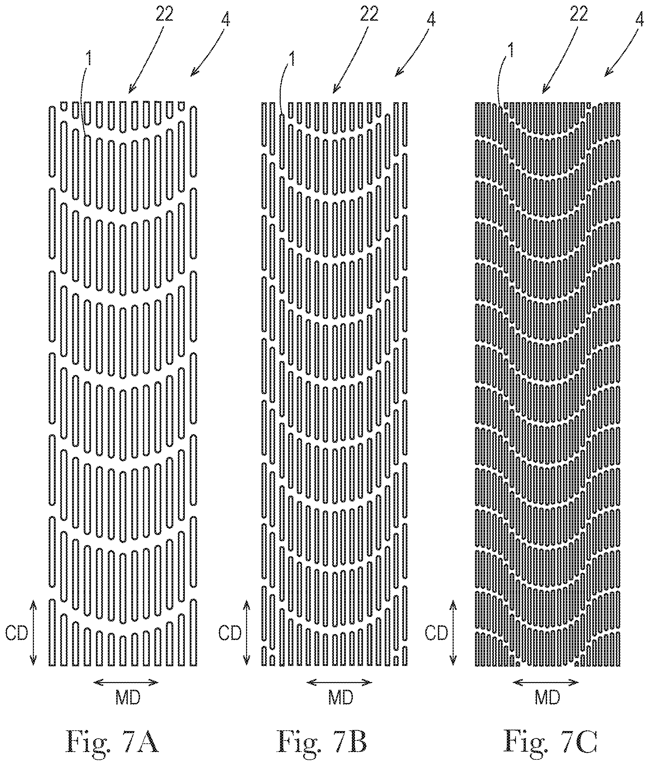

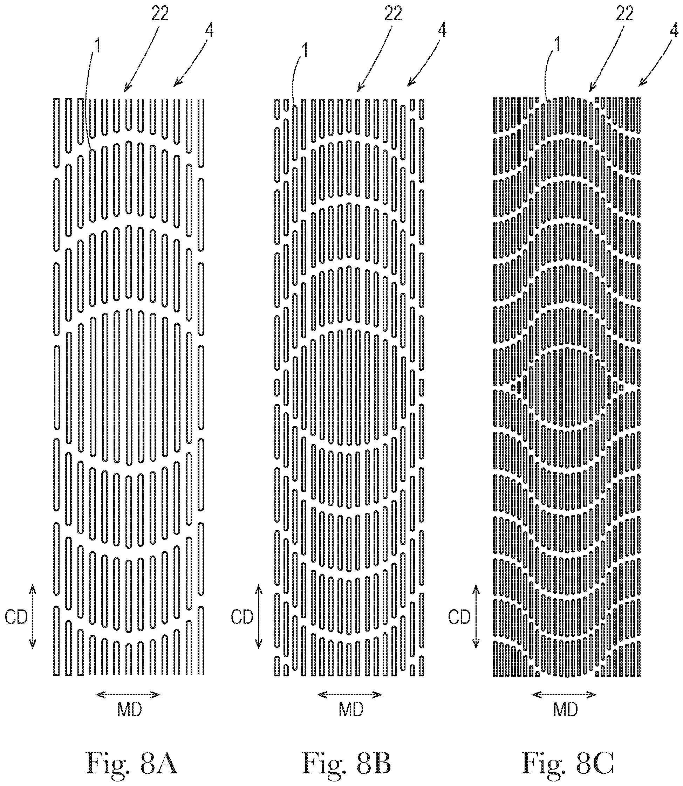

FIG. 6-8 are top views of potential patterns that may be created in the heterogeneous mass using formation means as described above. As shown in the FIG. 6, the canals 1 may be discontinuous such that the foam is continuous along the CD 2 and MD 3 directions. FIG. 7A-C and FIG. 8A-C represent additional optional patterns.

### Acquisition Speed

The SABAP (Speed of Acquisition with Balloon Applied Pressure) test is designed to measure the speed at which 0.9% saline solution is absorbed into an absorbent article which is compressed at 2.07 kPa. A known volume is introduced four times, each successive dose starting five (5) minutes after the previous dose has absorbed. Times needed to absorb each dose are recorded. All testing is performed in a room maintained at about 23 °C ± 2 C° and about 50% ± 2 % relative humidity. Samples are conditioned under the same conditions twelve (12) hours prior to testing.

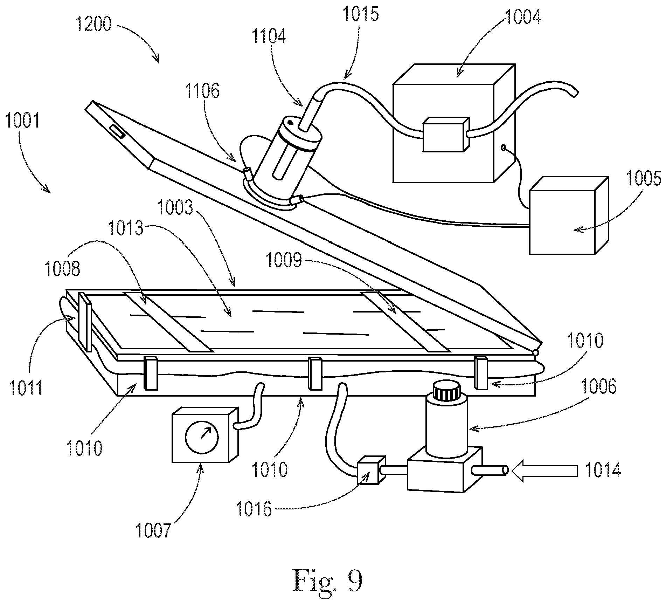

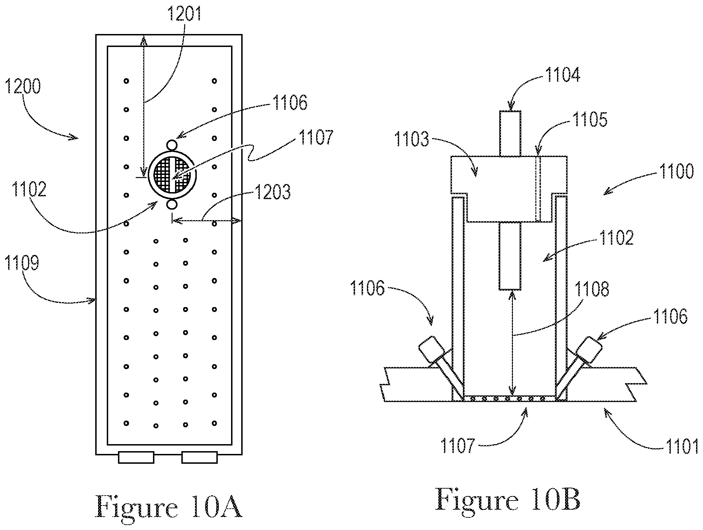

The SABAP apparatus is depicted in FIGS. 9 and 10A-B. It consists of a bladder assembly **1001** and atop plate assembly **1200** which includes the deposition assembly **1100.** A controller **1005** is used to 1) monitor the impedance across the electrodes **1106,** recording the time interval 0.9% saline solution is in the cylinder **1102,** 2) interface with the liquid pump **1004** to start/stop dispensing, and 3) time intervals between dosing. The controller is capable of recording time events to ± 0.01 sec. A house air supply **1014** is connected to the pressure regulator **1006** capable of delivering air at a suitable flow/pressure to maintain 2.07 kPa in the bladder assembly. A liquid pump **1004** (Ismatec MCP-Z gear pump, available from Cole Palmer, Vernon Hills, IL or equivalent) capable of delivering a flow of 10-80 mL at a rate of 3-15 mL/s is attached to the steel tube **1104** of the deposition assembly **1100** via Tygon tubing **1015.**

The bladder assembly **1001** is constructed of 12.7 mm Plexiglas with an overall dimension of 80 cm long by 30 cm wide by 5 cm tall. A manometer **1007** to measure the pressure inside the assembly and a pressure gauge **1006** to regulate the introduction of air into the assembly are installed through two holes through the right side. The bladder **1013** is assembled by draping a 50 mm by 100 mm piece of silicone film, (thickness 0.02", Shore A durometer value of 20, available as Part# 86435K85 from McMaster-Carr, Cleveland, OH) over the top of the box with enough slack that the latex touches the bottom of the box at its center point. An aluminum frame **1003** with a flange is fitted over the top of the latex and secured in place using mechanical clamps 1010. When in place the assembly should be leak free at a pressure of 3.45 kPa. A front **1008** and back **1009** sample support 5 cm by 30 cm by 1 mm are used to anchor the sample. The article is attached to the top surface of the sample supports by either adhesive tape or mechanical "hook" fasteners. These supports can be adjusted along the length of the aluminum frame **1003** via a simple pin and hole system to accommodate different size absorbent articles and to correctly align their loading point.

The top plate assembly **1200** is constructed of an 80 cm by 30 cm piece of 12.7 mm Plexiglas reinforced with an aluminum frame **1109** to enhance rigidity. The deposition assembly **1100** is centered 30 cm **(1201)** from the front of the plate assembly and 15 cm **(1203)** from either side. The deposition assembly is constructed of a 50.8 mm O.D. Plexiglas cylinder **1102** with a 38.1 mm I.D. The cylinder is 100 mm tall and is inserted through the top plate **1101** and flush with the bottom of the plate **1101.** Two electrodes **1106** are inserted though the top plate and cylinder and exit flush with the inner wall of the cylinder immediately above the cylinders bottom surface. A nylon screen **1107** cut into two semicircles are affixed flush with the bottom of the cylinder such that the sample cannot swell into the cylinder. The cylinder is topped with a loose-fitting nylon cap **1103.** The cap has a 6.35 mm O.D. steel tube **1104** inserted through its center. When the cap is in place, the bottom of the tube ends 20 mm above **(1108)** the screen **1107.** The cap also has an air hole **1105** to ensure negative pressure does not impede the absorption speed. In addition, the top plate has forty-four (44) 3.2 mm diameter holes drilled through it distributed as shown in Figure 10. The holes are intended to prevent air from being trapped under the top plate as the bladder is inflated but not to allow fluid to escape. The top plate assembly **1200** is connected to the bladder assembly 1001 via two hinges **1012.** During use the top assembly is closed onto the bladder assembly and locked into place using a mechanical clamp **1011.**

Samples are conditioned at 23 °C ± 2 C° and about 50% ± 2 % relative humidity for twelve (12) hours prior to testing. A sample article is prepared by placing the product flat onto a lab bench and identifying the intersection of the longitudinal and lateral centerlines of the article. For a pant product any cuffs or waistbands are remove taking care not to damage the top sheet or absorbent body of the article. Attach the front end of the article to the top surface of the front sample plate **1008** by either adhesive tape or mechanical "hook" fasteners with the top sheet facing upward. The placement is such that just the chassis and not the absorptive core overlays the plate. The sample plate **1008** is attached to the aluminum frame **1003** such that the absorbent article will be centered longitudinally and laterally within the cylinder **1102** when the top plate assembly has been closed. The back end of the article is secured to the back sample plate **1009** by either adhesive tape or mechanical "hook" fasteners, once again ensuring that only the chassis and not the absorptive core overlays the plate. The back sample plate **1009** is then attached to the aluminum frame **1003** such that the article is taunt but not stretched. The top plate assembly is closed and fastened, and the bladder is inflated to 2.07 kPa ± 0.07 kPa.

0.9% w/v saline solution is prepared by weighing 9.0 g ± 0.05g of NaCl into a weigh boat, transferring it into a 1L volumetric flask and diluting to volume with de-ionized water. The pump **1004** is primed then calibrated to deliver 20 mL at 5 mL/sec. Volume and flow rate must be within ± 2% of target. The cap **1103** is placed into the cylinder **1102.** The controller **1005** is started, which in turn delivers the first dose of 0.9% saline solution. After the volume has been absorbed, the controller waits for 5.0 minutes before addition of the next dose. This cycle is repeated for a total of four doses. If the fluid leaks out of or around the product (i.e., is not absorbed into the article) then the test is aborted. Also if any acquisition time exceeds 1200 sec, the test is aborted. Acquisition times are recorded by the controller for each dose to the nearest 0.01 sec. After the test is complete (i.e., 5 min after the last dose is absorbed), the pressure relief valve **1016** is opened to deflate the bladder and the sample article removed.

In like fashion run a total of five (5) replicates for each article to be evaluated. Calculate and report the acquisition rates mL/sec for each dose as the geometric mean to the nearest 0.01 mL/sec. Using the caliper from the Dry Caliper method described herin calculate the Acquisition Rate (mL/sec) divided by the Initial Caliper (mm) and report to the nearest 0.1 mL/sec/mm.

## Claims

1. An absorbent product (10) comprising a topsheet (14), a backsheet (16), and an absorbent core structure (18), the absorbent core structure comprising a top core layer (20) and a lower core level (30),
wherein the top core layer (20) comprises a heterogeneous mass layer comprising a longitudinal axis, a lateral axis, a vertical axis, and a HIPE foam (25) intermixed with a nonwoven web (44, 50),
wherein the heterogeneous mass contains stripes (26) of high capillarity and one or more canals (1) of high permeability,
wherein the stripes of high capillarity contain the HIPE foam and the nonwoven web; wherein the canals of high permeability contain between 0% and 40% of the amount of HIPE foam in the stripes of high capillarity and nonwoven web; and
wherein the lower core level comprises a substrate plus superabsorbent polymer layer containing greater than 50%, of a superabsorbent polymer and less than 30% of cellulose.

2. The absorbent product of claim 1, wherein the stripes are parallel to the longitudinal axis.

3. The absorbent product of claim 1, wherein the stripes are parallel to the lateral axis.

4. The absorbent product of claim 1, wherein the stripes are parallel to a diagonal axis.

5. The absorbent product of any of the preceding claim s, wherein one or more additional absorbent layers are combined with the top and lower level of the absorbent core structure.

6. The absorbent product of any of the preceding claims, wherein the canals are parallel between 30% to 100% of the length of the longitudinal axis, the lateral axis, or a diagonal dimension of the heterogeneous mass upper layer.

7. The absorbent product of any of the preceding claims, wherein the absorbent core structure exhibits a third acquisition time of less than 20 seconds, as measured by the SABAP test described herein.

8. The absorbent product of any of the preceding claims, wherein the absorbent core structure exhibits a fourth acquisition time of less than 20 seconds, as measured by the SABAP test described herein.

## Patentansprüche

1. Absorptionsprodukt (10), umfassend eine Oberschicht (14), eine Unterschicht (16) und eine Absorptionskernstruktur (18), wobei die Absorptionskernstruktur eine obere Kernschicht (20) und eine untere Kernebene (30) umfasst,
wobei die obere Kernschicht (20) eine heterogene Massenschicht umfasst, die eine Längsachse, eine Querachse, eine vertikale Achse und einen HIPE-Schaum (25) umfasst, der mit einer Vliesbahn (44, 50) vermischt ist,
wobei die heterogene Masse Streifen (26) von hoher Kapillarität und einen oder mehrere Kanäle (1) von hoher Permeabilität enthält,
wobei die Streifen von hoher Kapillarität den HIPE-Schaum und die Vliesbahn enthalten; wobei die Kanäle von hoher Permeabilität zwischen 0 % und 40 % der Menge an HIPE-Schaum in den Streifen von hoher Kapillarität und der Vliesbahn enthalten; und
wobei die untere Kernebene ein Substrat plus eine Superabsorberpolymerschicht umfasst, die mehr als 50 % eines Superabsorberpolymers und weniger als 30 % Cellulose enthält.

2. Absorptionsprodukt nach Anspruch 1, wobei die Streifen parallel zu der Längsachse sind.

3. Absorptionsprodukt nach Anspruch 1, wobei die Streifen parallel zu der Querachse sind.

4. Absorptionsprodukt nach Anspruch 1, wobei die Streifen parallel zu einer diagonalen Achse sind.

5. Absorptionsprodukt nach einem der vorstehenden Ansprüche, wobei eine oder mehrere zusätzliche Absorptionsschichten mit der oberen und der unteren Ebene der Absorptionskernstruktur verbunden sind.

6. Absorptionsprodukt nach einem der vorstehenden Ansprüche, wobei die Kanäle zwischen 30 % bis 100 % der Länge der Längsachse, der Querachse oder einer diagonalen Abmessung der oberen Schicht der heterogenen Masse parallel sind.

7. Absorptionsprodukt nach einem der vorstehenden Ansprüche, wobei die Absorptionskernstruktur eine dritte Aufnahmezeit von weniger als 20 Sekunden aufweist, wie durch den hierin beschriebenen SABAP-Test gemessen.

8. Absorptionsprodukt nach einem der vorstehenden Ansprüche, wobei die Absorptionskernstruktur eine vierte Aufnahmezeit von weniger als 20 Sekunden aufweist, wie durch den hierin beschriebenen SABAP-Test gemessen.

## Revendications

1. Produit absorbant (10) comprenant une feuille de dessus (14), une feuille de fond (16), et une structure d'âme absorbante (18), la structure d'âme absorbante comprenant une couche d'âme supérieure (20) et un niveau d'âme inférieur (30),
dans lequel la couche d'âme supérieure (20) comprend une couche de masse hétérogène comprenant un axe longitudinal, un axe latéral, un axe vertical, et une mousse HIPE (25) entremêlée avec une nappe non tissée (44, 50),
dans lequel la masse hétérogène contient des rayures (26) de capillarité élevée et un ou plusieurs canaux (1) de perméabilité élevée,
dans lequel les rayures de capillarité élevée contiennent la mousse HIPE et la nappe non tissée ; dans lequel les canaux de perméabilité élevée contiennent entre 0 % et 40 % de la quantité de mousse HIPE dans les rayures de capillarité élevée et la nappe non tissée ; et
dans lequel le niveau d'âme inférieur comprend une couche de substrat plus polymère superabsorbant contenant plus de 50 %, d'un polymère superabsorbant et moins de 30 % de cellulose.

2. Produit absorbant selon la revendication 1, dans lequel les rayures sont parallèles à l'axe longitudinal.

3. Produit absorbant selon la revendication 1, dans lequel les rayures sont parallèles à l'axe latéral.

4. Produit absorbant selon la revendication 1, dans lequel les rayures sont parallèles à un axe diagonal.

5. Produit absorbant selon l'une quelconque des revendications précédentes, dans lequel une ou plusieurs couches absorbantes supplémentaires sont combinées avec le niveau supérieur et inférieur de la structure d'âme absorbante.

6. Produit absorbant selon l'une quelconque des revendications précédentes, dans lequel les canaux sont parallèles entre 30 % et 100 % de la longueur de l'axe longitudinal, de l'axe latéral, ou d'une dimension diagonale de la couche supérieure de masse hétérogène.

7. Produit absorbant selon l'une quelconque des revendications précédentes, dans lequel la structure d'âme absorbante présente un troisième temps de recueil inférieur à 20 secondes, tel que mesuré par le test SABAP décrit ici.

8. Produit absorbant selon l'une quelconque des revendications précédentes, dans lequel la structure d'âme absorbante présente un quatrième temps de recueil inférieur à 20 secondes, tel que mesuré par le test SABAP décrit ici.
